# EUROPÄISCHE PATENTSCHRIFT

(11) **EP 1 627 693 B1**
(45) Veröffentlichungstag und Bekanntmachung des Hinweises auf die Patenterteilung: **16.11.2011**
(21) Anmeldenummer: 05015825.2
(22) Anmeldetag: 21.07.2005
(51) Int. Cl.: B08B 9/02, A61B 19/00, A61L 2/18, A61L 2/26

(54) **Vorrichtung zum Reinigen und/oder Desinfizieren von langgestreckten Hohlkörpern, insbesondere von medizinischen Schläuchen und Kathetern**
Device for cleaning and/or disinfecting elongated hollow bodies, in particular medical tubes and catheters
Dispositif de nettoyage et/ou de désinfection des corps creux, en particulier des tubes médicaux et des cathéters

(30) Priorität: 20.08.2004 DE 102004040734
(43) Veröffentlichungstag der Anmeldung: 22.02.2006
(73) Patentinhaber: BHT Hygienetechnik GmbH, 86368 Gersthofen (DE)
(72) Erfinder: Annecke, Karl Heinz, Dr., 64625 Bensheim (DE)
(74) Vertreter: von Bülow, Tam

(56) Entgegenhaltungen:
- EP-A- 0 075 657
- EP-A- 0 709 056
- EP-A- 1 502 538
- DE-A1- 2 751 743
- DE-A1- 3 720 347
- DE-A1- 4 217 577
- DE-B3- 10 321 991
- US-A- 3 875 955

## Beschreibung

Die Erfindung bezieht sich auf eine Vorrichtung zum Reinigen und/oder Desinfizieren von langgestreckten Hohlkörpern, insbesondere von medizinischen Schläuchen, Kathetern und Endoskopen gemäß dem Oberbegriff des Patentanspruches 1.

### Stand der Technik

Eine solche Vorrichtung ist aus der DE 103 21 991 B3 bekannt. Dort werden die zu reinigenden Gegenstände jeweils über eine Kupplung an einen Wasserkanal angeschlossen, wobei in Strömungsrichtung vor der Kupplung eine einstellbare Strömungsverengung und ein Drucksensor mit Speicherfunktion angeordnet sind. Bei ausreichendem Durchfluß von Reinigungsflüssigkeit durch den Hohlkörper tritt an der Strömungsverengung ein Druckabfall auf. Bei ganz oder teilweise verstopftem Hohlkörper baut sich dagegen ein Staudruck auf, der von dem Drucksensor erfaßt und dauerhaft gespeichert wird. Dadurch kann nach Beendigung eines Reinigungsvorganges an dem gespeicherten Wert sofort erkannt werden, ob ein oder mehrere Hohlkörper ordnungsgemäß durchspült und damit gereinigt wurden.

Aus der DE 296 20 011 U1 ist es bekannt, zum Reinigen mehrerer Katheter oder Schläuche an einem Wasserkanal mehrere Aufsteckdüsen anzubringen, die eine unlösbar mit ihr verbundene Befestigungseinrichtung aufweisen, die die Außenseite des zu reinigenden Gegenstandes form- oder kraftschlüssig halten. Diese Aufsteckdüsen dienen als Kupplung zur Verbindung des Schlauches mit dem Wasserkanal.

Zur Validierung des Reinigungs- und/oder Desinfektionsergebnisses von Endoskopen schlägt die DE 102 08 035 A1 vor, jedem Kanal einen Flüssigkeitsbehälter mit einem vorbestimmten Volumen zuzuordnen und zu überprüfen, ob dieses Volumen innerhalb vorgegebener Zeit durch den Kanal geflossen ist.

Die EP 0 711 529 A1 beschreibt ein Verfahren zum Prüfen und Reinigen von Endoskopen, bei dem jeder Fluidkanal über einen Verteiler mit Ventilen an eine Fluidquelle anschließbar ist. Mittels eines Durchflußprüfgerätes wird die Durchlässigkeit der Kanäle einzeln oder in Gruppen nacheinander geprüft, indem den Kanälen Fluid aus der Fluidquelle zugeführt wird. Zur Überprüfung der Durchlässigkeit wird überprüft, ob ein bestimmtes Fluidvolumen in einer bestimmten Zeiteinheit durch den jeweiligen Kanal geleitet wird. Die Messung der Durchflußmenge pro Zeiteinheit erfolgt dadurch, daß das Fluid unter Druck durch den Kanal gepreßt wird und der zeitliche Verlauf des Druckabfalls ausgewertet wird, woraus sich das durchströmte Volumen bestimmen läßt. Aufgrund des ermittelten Volumens pro Zeiteinheit soll dann das spezifische Endoskop identifiziert werden, indem der gemessene Wert mit gerätespezifischen Werten verglichen wird.

Die DE 36 01 395 A1 beschreibt eine Vorrichtung zur Anzeige anormaler Betriebszustände bei einer Endoskopwaschmaschine. Mit einer Vielzahl von Sensoren werden bestimmte Parameter, wie z.B. Pegelstände von Reinigungsflüssigkeit, gemessen bzw. überwacht und daraus ermittelt, ob eine Störung oder ein anormaler Betriebszustand vorliegen.

Die Kanäle von Endoskopen haben je nach Anwendungszweck (z.B. Arbeitskanal, Probenahmekanal, Injektionskanal etc.) sehr unterschiedliche Durchmesser. Weiter sind diese Kanäle sehr druckempfindlich, d.h. ein Fluiddruck in einem Kanal oberhalb vorbestimmter Grenzwerte kann zu einer Beschädigung des gesamten Endoskopes führen.

Bei der Prüfung der Kanäle auf Durchlässigkeit darf daher bei der eingangs genannten Vorrichtung gemäß DE 103 21 991 B3 der Druck der einzelnen Kanäle bestimmte Grenzwerte nicht überschreiten. Dies wird dadurch erreicht, daß der von einer Pumpe gelieferte Vordruck begrenzt wird und/oder dadurch, daß die den einzelnen Kanälen zugeordneten Düsen für das jeweilige Endoskop und den jeweiligen Kanal ausgelegt werden. Somit ist dort jede Kupplung nur für ein bestimmtes Endoskop mit einer bestimmten Solldurchflußmenge ausgelegt.

Bei der Prüfung verschiedener Endoskope oder Katheter mit verschiedenen Solldurchflußmengen oder bei der Prüfung verschiedener Endoskopkanäle ist es daher erforderlich, die Düsen und den jeweiligen Druckmesser mit Speicherfunktion den aktuellen Verhältnissen anzupassen, was in der Praxis sehr aufwendig ist, da die Düsen und die Druckmesser ausgewechselt werden müssen.

### Aufgabenstellung

Aufgabe der vorliegenden Erfindung ist es, die bekannte Vorrichtung dahingehend zu verbessern, daß unterschiedliche Hohlkörper mit ein und derselben Vorrichtung überprüft werden können.

Diese Aufgabe wird durch die in den Patentansprüchen 1 oder 2 angegebenen Merkmale gelöst. Vorteilhafte Ausgestaltungen und Weiterbildungen der Erfindung sind den Unteransprüchen zu entnehmen.

Das Grundprinzip der Erfindung besteht darin, jeder Kupplung wirkungsmäßig eine einstellbare Düse zuzuordnen, die für jede Kupplung für einen Hohlkörper aus mindestens zwei Düsen besteht, wobei jeder Düse ein steuerbares Absperrventil zugeordnet. Damit können pro Hohlkörper wahlweise eine oder mehrere Düsen aktiv geschaltet werden und somit bei konstantem Speisedruck der Pumpe unterschiedliche Drücke an dem jeweiligen Hohlkörper eingestellt werden. Die Düsen sind wirkungsmäßig parallel geschaltet. Ihre Ausgänge führen zu dem Druckmesser, der vorzugsweise eine elektrischer Druckmesser mit nachgeschaltetem elektronischem Speicher ist.

Nach einer anderen Variante der Erfindung sind jeder Kupplung für einen Hohlkörper ebenfalls mindestens zwei Düsen zugeordnet, wobei aber mehreren Düsen unterschiedlicher Kupplungen jeweils nur ein gemeinsames Absperrventil zugeordnet ist.

Vorzugsweise sind die Düsenquerschnitte im Zweierpotenzsystem (Binärsystem) ansteigend ausgelegt. Bei Verwendung von zwei Düsen mit Querschnitten von "1" und "2" lassen sich damit effektive Düsenquerschnitte von "1", "2" oder "3" realisieren. Bei Verwendung von einer Anzahl "n" von Düsen, können 2ⁿ verschiedene effektive Düsenquerschnitte realisiert werden. Beispielsweise können bei vier Düsen 2⁴ = 16 verschiedene Düsenquerschnitte eingestellt werden.

Zur Anpassung an die individuellen Eigenschaften eines Katheters oder Endoskopkanales können damit folgende drei Parameter variiert werden:
1. der effektive Düsenquerschnitt,
2. die Ansprechschwelle des Druckmessers mit elektronischem Speicher und
3. der Speisedruck P₀.

Durch diese Maßnahmen kann mit einem einzigen Aufbau die Funktionsweise der Vorrichtung so eingestellt werden, daß jeder Katheter bzw. jeder Endoskopkanal mit der optimalen Durchflußmenge bezüglich seiner Durchgängigkeit überprüft wird, der Druckmesser mit Speicherfunktion anspricht, wenn diese Durchflußmenge um einen vorprogrammierten Prozentsatz nach unten von der Sollmenge abweicht, was sich durch eine entsprechende Drucksteigerung bemerkbar macht. Der Druckmesser kann als Druckschalter ausgelegt sein, der bei einem programmierbaren Grenzwert umschaltet.

### Ausführungsbeispiel

Im folgenden wird die Erfindung anhand eines Ausführungsbeispieles im Zusammenhang mit der Zeichnung ausführlicher erläutert. Es zeigt:
Fig. 1 eine Prinzipskizze einer Vorrichtung nach einem ersten Ausführungsbeispiel der Erfindung; und
Fig. 2 eine Prinzipskizze einer Vorrichtung nach einem zweiten Ausführungsbeispiel der Erfindung.

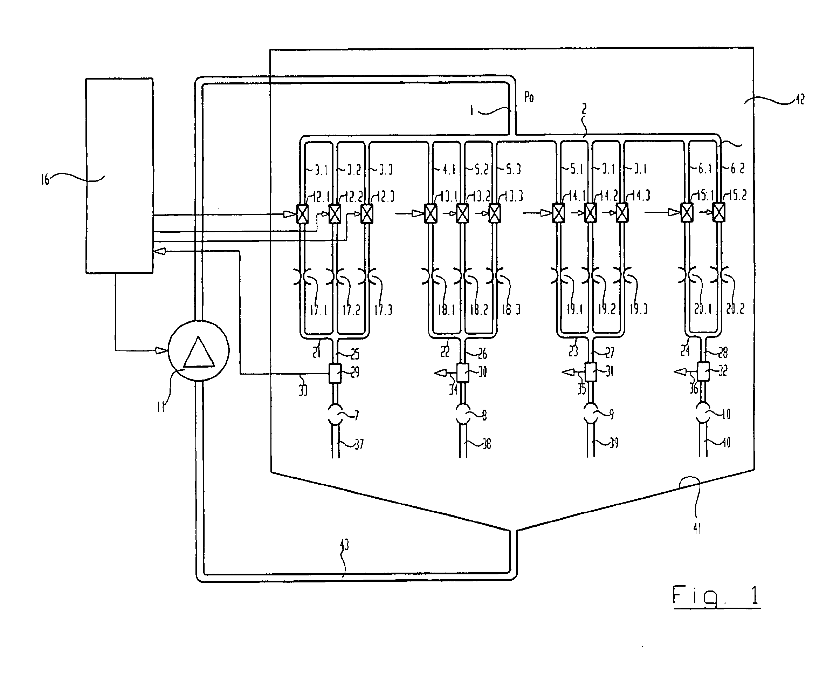

Fig. 1 zeigt eine Rohrleitung 1, die über einen Rohrverteiler 2 zu einer Vielzahl von Wasserkanälen 3.1, 3.2, 3.3 ... 6.1, 6.2 verzweigt, wobei mehrere Wasserkanäle, z.B. 3.1, 3.2, 3.3 oder 4.1, 4.2, 4.3 je eine Gruppe bilden, die jeweils einer Kupplung 7, 8, 9 oder 10 zugeordnet ist. Alle Wasserkanäle 3.1 bis 6.2 werden von der Rohrleitung 1 und dem Rohrverteiler 2 mit Flüssigkeit gespeist, die von einer Umwälzpumpe 11 mit einem vorgegebenen Druck P₀ geliefert wird. In jedem Wasserkanal 3.1 bis 6.2 befindet sich ein steuerbares Ventil 12.1, 12.2, 12.3, 13.1, 13.2, 13.3, 14.1, 14.2, 14.3 bzw. 15.1, 15.2. Jedes dieser Ventile ist einzeln von einer Steuerung 16 ansteuerbar und kann die Zustände "offen" oder "geschlossen" einnehmen, je nach Steuersignal auf einer entsprechenden elektrischen Steuerleitung. Weiter ist in jedem Wasserkanal 3.1 bis 6.2 eine Düse 17.1, 17.2, 17.3; 18.1, 18.2, 18.3; 19.1, 19.2, 19.3; 20.1, 20.2 angeordnet. Stromabwärts der Düsen sind die Rohrleitungen jeder Gruppe über einen Rohrverbinder 21, 22, 23 bzw. 24 miteinander verbunden, so daß sie gruppenweise dann in jeweils eine einzige Rohrleitung 25, 26, 27 bzw. 28 münden. In jede dieser letzt genannten Rohrleitungen 25, 26, 27, 28 ist je ein Drucksensor 29, 30, 31 bzw. 32 eingeschaltet, der über eine elektrische Leitung 33, 34, 35 bzw. 36 ein Drucksignal an die Steuerung 16 abliefert, das dort gespeichert wird.

Stromabwärts der Drucksensoren 29-32 sind dann die Kupplungen 7, 8, 9 bzw. 10 angeordnet, an die je ein Hohlkörper 37, 38, 39 bzw. 40 anschließbar ist. Der Hohlkörper kann ein Kanal eines Endoskopes, ein Katheter, ein Schlauch oder ähnliches sein. Die aus dem freien Ende der Hohlkörper 37-40 austretende Flüssigkeit gelangt in einen Sumpf 41 eines Gehäuses 42 einer Reinigungsmaschine und wird von dort über eine Rohrleitung 43 zur Umwälzpumpe 11 zurückgeführt.

Zum Einstellen des Prüfdruckes der durch die Hohlkörper 37, 38, 39 bzw. 40 fließenden Flüssigkeit können die einzelnen Düsen 17.1 bis 20.2 zu- oder abgeschaltet werden. Da diese gruppenweise parallel liegen, kann man selektiv den wirksamen Düsenquerschnitt gezielt einstellen. Von besonderem Vorteil ist es, wenn die Düsen einer Gruppe, wie z.B. die Düsen 17.1, 17.2 und 17.3, unterschiedlichen Querschnitt haben. Bevorzugt ist es dabei noch, wenn die Stufung nach dem Binärsystem erfolgt, also beispielsweise die Düse 17.1 einen Querschnitt von "1" hat, die Düse 17.2 den Querschnitt "2" und die Düse 17.3 den Querschnitt "4". Bei n-Düsen erhält man damit 2ⁿ Kombinations- und damit Abstufungsmöglichkeiten. Die oben genannten Werte von "1", "2", "4" sind dabei relative Werte, die auf einen vorbestimmten Düsenquerschnitt normiert sind.

Für jede der genannten Gruppen, d.h. für jeden der Hohlkörper 37, 38, 39 und 40 läßt sich somit individuell über die Ventile 12.1 bis 15.2 ein Prüfdruck einstellen, der auf den Ausgangsdruck P⁰ der Umwälzpumpe 11 bezogen ist. Auch dieser Druck läßt sich von der Steuerung 16 einstellen, beispielsweise durch Änderung der Drehzahl der Umwälzpumpe 11. Schließlich lassen sich auch Grenzdrücke für die Drucksensoren 29, 30, 31 und 32 einstellen. Hierbei kann vorgesehen sein, daß bei Überschreiten eines solchen voreingestellten Druckes, was ein Indikator für einen nicht oder nicht korrekt durchlässigen Hohlkörper ist, die Umwälzpumpe 11 abgeschaltet wird, um einen unzulässig hohen Druck im jeweiligen Hohlkörper zu vermeiden. Zusätzlich wird der maximale Druck der Drucksensoren 29-32 gespeichert, was als Nachweis für eine einwandfreie Durchgängigkeit der Hohlkörper dient.

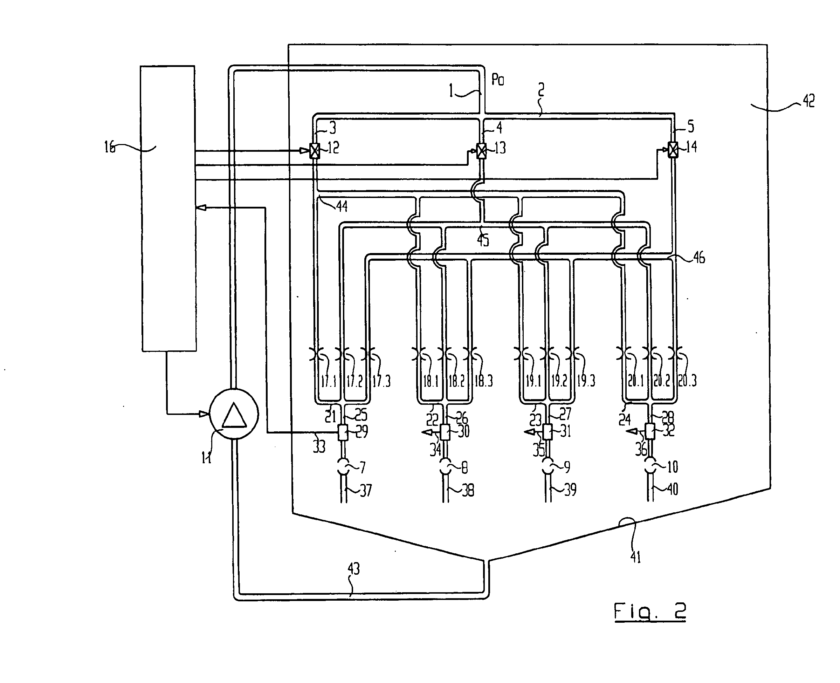

Fig. 2 zeigt eine Variante der Erfindung, die sich von der der Fig. 1 im wesentlichen dadurch unterscheidet, daß jeweils ein Absperrventil mehreren Düsen zugeordnet ist. Diese Variante geht davon aus, daß beispielsweise bei großen Endoskopen alle Kanäle einen größeren Durchmesser haben und bei kleinen Endoskopen alle einen kleineren Durchmesser. Im einzelnen sind in Fig. 2 an den Rohrverteiler 2 drei Leitungen 3, 4 und 5 angeschlossen, in die jeweils ein steuerbares Absperrventil 12, 13 bzw. 14 eingesetzt ist. An die Ausgänge dieser Ventile 12, 13 und 14 ist je ein Rohrverteiler 44, 45 bzw. 46 angeschlossen. An den Rohrverteiler 44 sind je eine Düse 17.1, 18.1, 19.1 und 20.1 jeder Gruppe von Düsen angeschlossen, die zu den einzelnen Kupplungen 7, 8, 9 und 10 führen. In ähnlicher Weise sind an den zweiten Rohrverteiler 45 die Düsen 17.2, 18.2, 19.2 und 20.2 angeschlossen und an den dritten Rohrverteiler 46 die Düsen 17.3, 18.3, 19.3 und 20.3.

Die Ausgänge aller Düsen sind - wie bei Fig. 1 - wiederum über Rohrverbinder 21, 22, 23 und 24 zu Rohrleitungen 25, 26, 27 und 28 zusammengefaßt, in die die Drucksensoren 29, 30, 31 und 32 eingesetzt sind. Durch Schalten eines oder mehrerer der Ventile 12, 13 oder 14 werden somit für alle Gruppen, d.h. für alle Kupplungen 7, 8, 9 und 10 die wirksamen Düsenquerschnitte gemeinsam umgeschaltet. Bei den hier gezeigten drei Düsen pro Gruppe ergeben sich damit 2³ =8 Stufungsmöglichkeiten.

Mit der Vorrichtung nach der Erfindung können ohne bauliche Änderungen viele unterschiedliche Hohlkörper gereinigt und geprüft werden, wobei jeweils in Abstimmung auf den jeweiligen Hohlkörper, wie z.B. ein Endoskop, die Drücke in der programmierbaren Steuerung, die beispielsweise ein Mikroprozessor ist, festgelegt werden.

Die Drucksensoren können beliebiger Bauart sein. Beispielsweise können sie gemäß der DE 103 21 991 B3 ausgebildet sein. Es können aber auch sonstige Drucksensoren verwendet werden, die im Stand der Technik bekannt sind. Dabei können es sowohl Drucksensoren mit eigener Speicherfunktion sein als auch normale Drucksensoren, die ein druckproportionales Ausgangssignal liefern, das dann in der Steuerung 16 gespeichert wird.

## Patentansprüche

1. Vorrichtung zum Reinigen und/oder Desinfizieren von Hohlkörpern, insbesondere von medizinischen Schläuchen, Kathetern oder Endoskopen mit mindestens einer an einen Wasserkanal angeschlossenen Kupplung zur Verbindung des Wasserkanales mit dem Hohlkörper, wobei in Strömungsrichtung vor der Kupplung eine Strömungsverengung in Form einer einstellbaren Düse und ein Druckensor angeordnet sind,
**dadurch gekennzeichnet,**
**daß** die einstellbare Düse durch mindestens zwei strom aufwärts vor der Kupplung (7, 8, 9, 10) angeordnete Strömungsverengungen (17.1, 17.2, 17.3; 18.1, 18.2, 18.3; 19.1, 19.2, 19.3; 20.1, 20.2) realisiert ist, die parallel zueinander angeordnet sind, und daß jeder Strömungsverengung ein steuerbares Absperrventil (12.1, 12.2, 12.3; 13.1, 13.2, 13.3; 14.1, 14.2, 14.3; 15.1, 15.2) zugeordnet ist.

2. Vorrichtung zum Reinigen und/oder Desinfizieren von Hohlkörpern, insbesondere von medizinischen Schläuchen, Kathetern oder Endoskopen, mit mindestens zwei an je einen Wasserkanal angeschlossenen Kupplungen zur Verbindung des jeweiligen Wasserkanales mit je einem Hohlkörper, wobei in Strömungsrichtung vor jeder Kupplung eine Strömungsverengung in Form einer einstellbaren Düse und ein Drucksensor angeordnet sind, **dadurch gekennzeichnet,**
**daß** jede einstellbare Düse durch mindestens zwei stromaufwärts vor der Kupplung (7, 8, 9, 10) angeordnete Strömungsverengungen (17.1, 17.2, 17.3; 18.1, 18.2, 18.3; 19.1, 19.2, 19.3; 20.1, 20.2) realisiert ist, die parallel zueinander angeordnet sind,
**daß** jeweils eine Strömungsverengung der ersten Düse mit jeweils einer Strömungsverengung der weiteren Düse(n) verbunden ist, und
**daß** den jeweils miteinander verbundenen Strömungsverengungen ein gemeinsames steuerbares Absperrventil (12, 13, 14) zugeordnet ist, wobei jedes der Absperrventile (12, 13, 14) über einen Rohrverteiler (44, 45, 46) mit den einzelnen Strömungsverengungen (17.1, 17.2, 17.3; 18.1, 18.2, 18.3; 19.1, 19.2, 19.3; 20.1, 20.2) verbunden ist.

3. Vorrichtung nach Anspruch 2 oder 3, **dadurch gekennzeichnet,**
**daß** die jeder Kupplung (7, 8, 9, 10) zugeordneten Strömungsverengungen (17.1, 17.2, 17.3; 18.1, 18.2, 18.3; 19.1, 19.2, 19.3; 20.1, 20.2) unterschiedliche Querschnitte haben.

4. Vorrichtung nach Anspruch 3, **dadurch gekennzeichnet, daß** die einer Kupplung (7, 8, 9, 10) zugeordneten Strömungsverengungen im Binärsystem gestuft sind.

5. Vorrichtung nach einem der Ansprüche 1 bis 4, **dadurch gekennzeichnet,**
**daß** die jeder Kupplung (7, 8, 9, 10) zugeordneten Drucksensoren (29, 30, 31, 32) Druckschalter sind, die bei einem vorbestimmten Druck umschalten.

6. Vorrichtung nach Anspruch 5, **dadurch gekennzeichnet, daß** der Umschaltdruck einstellbar ist.

7. Vorrichtung nach einem der Ansprüche 1 bis 6, **dadurch gekennzeichnet,**
**daß** der Wasserkanal (1) an eine Umwälzpumpe (11) angeschlossen ist, deren Ausgangsdruck (P₀) von einer Steuerung (16) einstellbar ist.

8. Vorrichtung nach Anspruch 7, **dadurch gekennzeichnet, daß** die Steuerung (16) die Umwälzpumpe (11) abschaltet, wenn ein Drucksensor (29, 30, 31, 32) einen Druck meldet, der höher als ein vorgegebener Wert ist.

## Claims

1. Device for cleaning and/or disinfecting of hollow bodies, in particular of medical tubes and catheters or endoscopes, having at least one coupling connected to a water channel, for connecting the water channel with the hollow body, wherein upstream of the coupling a flow constriction in form of a adjustable nozzle and a pressure sensor are arranged,
**characterized in that**
the adjustable nozzle is realized by at least two flow constrictions (17.1, 17.2, 17.3; 18.1, 18.2, 18.3; 19.1, 19.2, 19.3; 20.1, 20.2) arranged upstream of the coupling (7, 8, 9, 10), said flow constrictions being arranged parallel to each other, and **in that** an adjustable block valve (12.1, 12.2, 12.3; 13.1, 13.2, 13.3; 14.1, 14.2, 14.3; 15.1, 15.2) is assigned to each flow constriction.

2. Device for cleaning and/or disinfecting of hollow bodies, in particular of medical tubes, catheters or endoscopes, having at least two couplings connected to a water channel, for connecting the water channel with the hollow body, wherein upstream of the coupling a flow constriction in form of a adjustable nozzle and a pressure sensor are arranged,
**characterized in that**
each adjustable nozzle is realized by at least two flow constrictions (17.1, 17.2, 17.3; 18.1, 18.2, 18.3; 19.1, 19.2, 19.3; 20.1, 20.2) arranged upstream of the coupling (7, 8, 9, 10), said flow constrictions being arranged parallel to each other,
**in that** a respective flow construction of the first nozzle is connected with a respective flow construction of the further nozzle(s), and
**in that** a common controllable block valve (12, 13, 14) is assigned to each flow constrictions which are connected to each other, wherein each block valve (12, 13, 14) is connected to the individual flow constrictions (17.1, 17.2, 17.3; 18.1, 18.2, 18.3; 19.1, 19.2, 19.3; 20.1, 20.2) via a pipe manifold (44, 45, 46).

3. Device according to claim 2 or 3, **characterized in that** the flow constrictions (17.1, 17.2, 17.3; 18.1, 18.2, 18.3; 19.1, 19.2, 19.3; 20.1, 20.2) assigned to each coupling (7, 8, 9, 10) have different cross sections.

4. Device according to claim 3, **characterized in that** the flow constrictions assigned to a coupling (7, 8, 9, 10) are graded in the binary system.

5. Device according to one of claims 1 to 4, **characterized in that**, that the pressure sensors (29, 30, 31) assigned to each coupling (7, 8, 9, 10) are pressure switches which switch at a predetermined pressure.

6. Device according to claim 5, **characterized in that** the switch pressure is adjustable.

7. Device according to one of claims 1 to 6, **characterized in that**
the water channel (1) is connected to a circulating pump (11), the outlet pressure (P₀) of which is adjustable by a control device (16).

8. Device according to claim 7, **characterized in that** the control device (16) switches off the regulating pump (11), when the pressure sensor (29, 30, 31, 32) notifies a pressure being higher than a predetermined value.

## Revendications

1. Dispositif de nettoyage et/ou de désinfection de corps creux, en particulier de tubes médicaux, de cathéters ou d'endoscopes, comportant au moins un couplage adjoint à un canal d'eau pour relier le canal d'eau au corps creux, dans lequel un étranglement de courant sous la forme d'une buse réglable et un capteur de pression sont disposés dans le sens du courant avant le couplage,
**caractérisé en ce que**
la buse réglable est réalisée par au moins deux étranglements de courant (17.1, 17.2, 17.3; 18.1, 18.2, 18.3; 19.1, 19.2, 19.3; 20.1, 20.2) disposés en amont du couplage (7, 8, 9, 10), qui sont disposés parallèlement l'un par rapport à l'autre, et **en ce qu'**à chaque étranglement de courant est affectée une soupape d'arrêt pouvant être commandée (12.1, 12.2, 12.3; 13.1, 13.2, 13.3; 14.1, 14.2, 14.3; 15.1, 15.2).

2. Dispositif de nettoyage et/ou de désinfection de corps creux, en particulier de tubes médicaux, de cathéters ou d'endoscopes, comportant au moins deux couplages raccordés respectivement à un canal d'eau pour relier le canal d'eau respectif à respectivement un corps creux, dans lequel un étranglement de courant sous la forme d'une buse réglable et un capteur de pression sont disposés dans le sens du courant avant le couplage,
**caractérisé en ce que**
chaque buse réglable est réalisée par au moins deux étranglements de courant (17.1, 17.2, 17.3; 18.1, 18.2, 18.3; 19.1, 19.2, 19.3; 20.1, 20.2) disposés en amont du couplage (7, 8, 9, 10), qui sont disposés parallèlement l'un par rapport à l'autre,
**en ce que**, respectivement, un étranglement de courant de la première buse est relié respectivement à un étranglement de courant de l'autre/des autres buses, et
**en ce qu'**aux étranglements de courant respectifs reliés l'un à l'autre est affectée une soupape d'arrêt commune pouvant être commandée (12, 13, 14), chacune des soupapes d'arrêt (12, 13, 14) étant reliée par un répartiteur de tube (44, 45, 46) aux étranglements de courant individuels (17.1, 17.2, 17.3; 18.1, 18.2, 18.3; 19.1, 19.2, 19.3; 20.1, 20.2).

3. Dispositif selon la revendication 2 ou 3, **caractérisé en ce que**
les étranglements de courant (17.1, 17.2, 17.3; 18.1, 18.2, 18.3; 19.1, 19.2, 19.3; 20.1, 20.2) affectés à chaque couplage (7, 8, 9, 10) ont des sections différentes.

4. Dispositif selon la revendication 3, **caractérisé en ce que**
les étranglements de courant affectés à un couplage (7, 8, 9, 10) sont en système binaire.

5. Dispositif selon l'une des revendications 1 à 4, **caractérisé en ce que**
les capteurs de pression (29, 30, 31, 32) affectés à chaque couplage (7, 8, 9, 10) sont des pressostats qui commutent à une pression prédéterminée.

6. Dispositif selon la revendication 5, **caractérisé en ce que**
la pression de commutation est réglable.

7. Dispositif selon l'une des revendications 1 à 6, **caractérisé en ce que**
le canal d'eau (1) est relié à une pompe de recirculation (11) dont la pression de sortie (P₀) est réglable par une commande (16).

8. Dispositif selon la revendication 7, **caractérisé en ce que**
la commande (16) arrête la pompe de recirculation (11) lorsqu'un capteur de pression (29, 30, 31, 32) signale que la pression est supérieure à une valeur prédéterminée.
